# EUROPEAN PATENT SPECIFICATION

(11) **EP 1 751 225 B1**
(45) Date of publication and mention of the grant of the patent: **05.08.2009**
(21) Application number: 05728997.7
(22) Date of filing: 18.03.2005
(51) Int. Cl.: C08L 1/28, A61K 47/38, A61K 9/00

(54) **THERMOPLASTIC FILMS AND METHODS FOR MAKING**
THERMOPLASTISCHE FILME UND HERSTELLUNGSVERFAHREN DAFÜR
FILMS THERMOPLASTIQUES ET PROCEDES DE FABRICATION

(30) Priority: 19.03.2004 US 805172; 16.02.2005 US 653670 P
(43) Date of publication of application: 14.02.2007
(73) Proprietor: The Tapemark Company, West St. Paul, MN 55118-4000 (US)
(72) Inventor: JANNUSCH, Leonard, C., White Bear Lake, MN 55110 (US); BUNNELLE, William, L., Andover, MN 55304 (US); HUME, Robert, M., III, Woodbury, MN 55125 (US)
(74) Representative: Alton, Andrew
(86) International application number: PCT/US2005/009203
(87) International publication number: WO 2005/090461

(56) References cited:
- EP-A- 0 250 187
- WO-A-99/55312
- GB-A- 565 489
- GB-A- 1 051 514
- US-A- 4 940 587

## Description

This application is being filed on 18 March 2005, as a PCT International Patent application in the name of The Tapemark Company, a U.S. national corporation, applicant for the designation of all countries except the US, and Leonard C. Jannusch, William L. Bunnelle, and Robert M. Hume III, all U.S. citizens, applicant for the designation of the US only, and claims priority to U.S. Utility Application No. 10/805,172, filed March 19, 2004, and U.S. Provisional Application No. Unknown, filed February 26, 2005.

### Field of the Disclosure

The present disclosure relates to dispersible polymeric films, including edible films, and methods of making these films. More specifically, this disclosure relates to dispersible thermoplastic films, including edible films.

### Background of the Disclosure

There are several dispersible film products available that provide various desired features such as breath freshening and other oral hygiene properties when placed in the mouth of the user. The term dispersible is used to mean that the film is configured to dissolve or break apart into small pieces. The film product is typically small, configured to fit on a person's tongue. The film product is sufficiently rigid to handle and delays softening until placed in the mouth. Upon exposure to saliva, the bulk of the film product disperses, releasing the functional ingredients. The term disperse is used to mean dissolve or break apart into small pieces.

The film compositions that are used to make these film products are often aqueous based, made from a polymeric material that is made into a solution with various ingredients such as gums, thickeners, flavorants, and the like, dissolved in water or a water and ethanol solvent. This film composition is typically cast onto a liner and then dried. Due to the high percentage of volatile substance in the film composition, the coated film cannot be quickly dried of water and any solvent. The drying process consumes considerable time and energy.

What is desired, therefore, is an acceptable film product that is more conducive to quick manufacturing.

WO 99/55312 describes a layered pharmaceutical delivery film for application of a pharmaceutical to mucosal surfaces. The device comprises an adhesive layer and a nonadhesive backing layer, and the pharmaceutical may be provided in either or both layers. The film forming polymer may be hydroxy propyl cellulose and the composition may include plasticizer, such as a fatty alcohol

US-4,940,587 describes an arrangement for the application of an oral pharmaceutical to the mucosa of the oral or nasal cavity, for sustained release of a drug, The arrangement comprises a body formed of granules of a higher aliphatic alcohol and a hydrated water soluble hydroxyalkyl cellulose having the drug distributed therethrough. This body is coated with a cellulose derivative which is adherent to the mucosa. The preferred cellulose derivative is hydroxypropyl cellulose, the preferred higher aliphatic alcohol is cetostearyl alcohol, and the preferred water soluble cellulose is hydroxyethyl cellulose.

EP-0250187 describes a bioadhesive extruded single or multi-layered thin film, useful in intra-oral controlled-releasing delivery, having a water soluble or swellable polymer matrix bioadhesive layer which can adhere to a wet mucous surface. The bioadhesive layer consists essentially of 40-95% by weight of a hydroxypropyl cellulose, 5-60% of a homopolymer of ethylene oxide, 0-10% of a water-insoluble polymer such as ethyl cellulose, propyl cellulose, polyethylene and polypropylene, and 2-10% of a plasticizer. The film can have a medicament incorporated therein.

The present invention is directed to a thermoplastic film that is dispersible when in contact with a body fluid. The film is biologically compatible, so that it is capable of being assimilated into the body. For some applications, the film is edible, and disperses upon contacting the film to the eater's tongue. The edible film can include a breath freshening agent or, medicament or a nutrient. For other applications, such as topical applications or in a mucous membrane, the film can include a medicament.

According to a first aspect of the invention, there is provided a film formed by the method including the steps of providing a water-soluble film former; thoroughly mixing the film former in a fatty alcohol component to form a mixture, the fatty alcohol component present at a percentage weight level of the total films sufficient to cause the film to change from a solid to a liquid within a relatively narrow temperature range of 20 °C or less; and extruding the mixture to form a coated film, wherein the mixture is at a temperature of at least 65 °C when extruded; so that when the coated film cools to 30 °C or lower, the extruded film is non-tacky.

According to a second aspect of the invention, there is provided a method of making a film, the method including the steps of: providing a water-soluble film former; thoroughly mixing the film former in a fatty alcohol component to form a mixture, the fatty alcohol component present at a percentage weight level of the total film sufficient to cause the film to change from a solid to a liquid within a relatively narrow temperature range of 20 °C or less; and extruding the mixture to form a coated film, wherein the mixture is at a temperature of at least 65°C when extruded.

According to a third aspect of the invention, there is provided A method of making a film, the method including the steps of providing a water-soluble film former; thoroughly mixing the film former with a fatty alcohol to form a mixture having less than 5 wt. % water, wherein the mixture comprises fatty alcohol at a percentage weight level of the total film sufficient to cause the film to change from a solid to a liquid within a relatively narrow temperature range of 20° C or less; and extruding the mixture to form a coated film, wherein the mixture is at a temperature of at least 65 °C when extruded.
FIG. 1 is a schematic depiction of a container having a plurality of film product pieces therein;
FIG. 2 is a perspective view of a single film product; and
FIG. 3 is a schematic diagram of a coating apparatus suitable for extruding the coated film of the present invention.

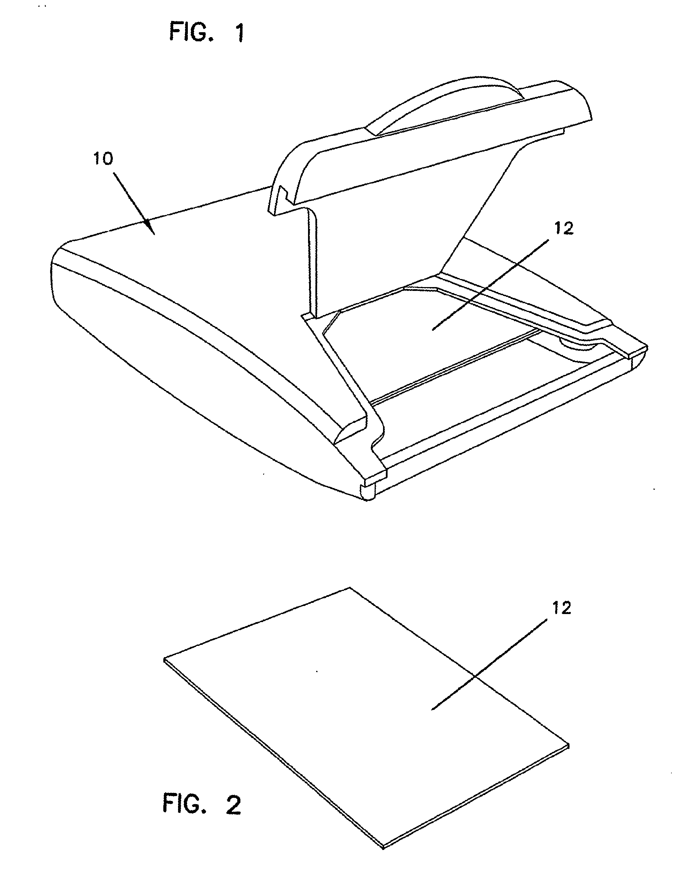

Referring to the figures, FIG. 1 illustrates a container 10 having a plurality of film product pieces 12 retained therein. A single film product 12 is illustrated in FIG. 2. In one embodiment, film product is designed to be orally ingested by a consumer. In other embodiment, film product is designed to disperse in a topical application on skin, or in contact with a mucous membrane, or in contact with a body fluid such as saliva or blood of a human or other animal.

Examples of conventional products similar to that illustrated in FIGS. 1 and 2 include"Listerine Cool Mint Pocket Paks", which are breath freshening strips from Pfizer Consumer Healthcare,"Chloraseptic Sore Throat Relief Strips", which are sore throat relief strips from Prestige Brands International, Inc. , and "Altoids Strips", which are breath freshening strips from Callard & Bowser-Suchard, Inc., and "Fresh Breath Strips", which are also breath freshening strips from Walgreen Co.

For the configurations illustrated in FIGS. 1 and 2, film product 12 is rectangular and has height and width dimensions of 1-4 cm and is 0.03 to 0.1 mm thick, usually 0.05 to 0.08 mm thick. A typical film product 12 weighs approximately 0.3 grams.

The term film refers to a thin flexible sheet of material and is intended to encompass the coated film, the cooled coated film and the film product. The film has a thickness sufficient to be rigid enough so that film product pieces with a perimeter area of 2-15 square cm are self-supporting, so that they do not bend under their own weight when grasped at one part, typically, at least 0.01 mm thick. The film typically has a thickness of no more than 2 mm, more typically no more than 1 mm. A thickness of 0.03 to 0.5 mm is preferred, depending on the use of film 12. For edible applications, a thickness of 0.03 to 0.1 mm is preferred.

In one embodiment, the film product has a perimeter area of 15 square cm or less, or, more preferably, 10 square cm or less. The film product preferably has a perimeter area of 4-8 square cm for edible films. Many different sizes for the film can be used, depending on the application.

The film product of the present invention can have many different shapes including rectangular, circular, oval, triangular, animal shapes or other fanciful shapes. The film products are often rectangular with a length and width at least 0.5 cm, usually at least 1 cm. The length and width are usually no greater than 30 cm, generally no greater than 20 cm. For edible applications, the length and width are usually 1 cm to 4 cm. In one particular embodiment, film product is 2.25 cm by 3 cm. In another particular embodiment the film product is 2 cm by 3 cm.

The film product in general, is thermoplastic, with adequate film integrity and rigidity to withstand handling. By use of the term "thermoplastic", what is intended is a polymer that softens and flows when exposed to a selected level of heat above room temperature and returns to its original rigid condition when cooled to a selected temperature, for example room temperature.

In addition, a thermoplastic material can be softened and solidified without undergoing an appreciable chemical change if the temperature of the material does not exceed some temperature, such as 275 °F (135 °C). For example, a thermoplastic material does not degrade or char when heated to a temperature of 135 °C or below.

Preferably, the film is solid at temperatures up to at least 100 °F (38 °C), more preferably it is solid up to at least 130 °F (54 °C). In one embodiment, the film begins to soften and flow at a temperature that is higher than 130 °F (54 °C). In a more preferred embodiment, the film begins to soften and flow at a temperature that is equal to or higher than 180 °F (82 °C), more preferably 200 °F (93 °C) or higher.

Preferably the film is configured to disperse upon exposure to water or a body fluid. In one embodiment, film pieces 12 are non-blocking so that they can be stacked on one another without sticking together at temperatures of up to 130°F (54 °C). In other embodiments, it is desirable for the film pieces to have an adhesive quality at room temperature so that they can stick to the skin or other surface.

Those film products intended for oral ingestion preferably have a pleasant or desirable taste. The film former, viscosity modifier, and other ingredients apart from any sweeteners or flavorants present in the film product can have a neutral taste that is not detectable to a consumer. Additionally, it is desired that the film disperses within a few minutes, preferably within 30 seconds, when exposed to saliva.

Film product pieces 12 of the present invention are made from a thermoplastic coatable composition. In accordance with the present invention, the coatable composition is a liquid mixture at a temperature higher than typical room temperatures. The composition is extruded or otherwise coated to form a coated film, and cools to room temperature to form a cooled coated film. This film can be cut into film product. The coatable composition of the present invention includes a thermoplastic film former, a viscosity modifier as a solvent, and any ingredients for tailoring the film for its intended use.

No more than 10 wt-% of the coatable composition is water or moisture based on the total weight of the coatable composition. In one embodiment, the water content in the coatable composition is generally no more than 6 wt-%, typically no more than 3 wt-%, and preferably no more than 2 wt-% based on the total weight of the composition. This level of water or moisture includes water than may be present in the ingredients. For example, moisture may be present in powdered ingredients. As another example, water may be the carrier for a flavor or dye. Preferably, there is no water added as liquid water to the coatable compositions. The resulting film has no more than 3 wt-% water, preferably no more than 2 wt-% water, and more preferably no more than 0.5 wt-% water based on the total weight of the coated film. In one embodiment there is no detectable level of water in the coated film.

The film product is not very sensitive to humidity changes, and is not likely to curl in response to changes in humidity. As a result, more materials are available for packaging the film pieces inexpensively.

In one embodiment, the film has dimensional stability and does not significantly expand in response to humidity changes. For example, in one embodiment, the film will expand or contract less than 30% and preferably less than 20% in any one dimension in response to a change in humidity. In another embodiment, the film will expand or contract less than 10% and preferably less than 5% in any one dimension in response to a change in humidity.

As stated above, the coatable composition is a thermoplastic composition. After forming the coatable composition, which is done in a liquid state to facilitate mixing of the ingredients, the composition can be immediately coated, or, the composition can be solidified for later coating. If storage of the composition for later coating is desired, the coatable composition can be solidified into blocks, bricks, pellets, or any other suitable form. The solid composition can be crushed, grated, or otherwise reduced in size to facilitate subsequent melting and coating.

### Examples of Film Uses

In one example of an application of the film product of the present invention, the film product is orally consumed for freshening breath or for delivering a substance to the consumer. In this application context, a film piece that is 0.05 mm thick, 2.25 cm wide and 3 cm long will preferably disperse when in contact with saliva in a consumer's mouth in less than 5 minutes, or more preferably less than 2 minutes. Preferably, the film is configured to disperse in contact with saliva within a time period less than 1 minute, or more preferably 30 seconds or less.

In another application, the film product can be a component of a bandage or dressing that retains an active ingredient in powder form in place in the bandage. When the film product contacts the blood or other fluid of the wound, it disperses, releasing the active ingredient to the wound. Alternatively, the film product can be applied to a wound to deliver an active ingredient to the wound site. In this application context, a film piece that is 0.05 mm thick, 2.25 cm wide and 3 cm long will preferably disperse when in contact with the blood or other fluid of the wound in a time period less than 5 minutes, or more preferably less than 2 minutes. Preferably, the film product is configured to disperse in use in contact with the body fluid within a time period less than 1 minute, or more preferably 30 seconds or less.

In another application example, the film product can be applied to skin to deliver an active ingredient that is beneficial to the skin. In order to release the active ingredient, the user rubs the film product with a wet finger moistened with saliva or water, causing the film product to disperse. In this application context, a film piece that is 0.05 mm thick, 2.25 cm wide and 3 cm long will preferably disperse when moistened in a time period less than 5 minutes, or more preferably less than 2 minutes. Preferably, the film product is configured to disperse within a time period less than 1 minute, or more preferably 30 seconds or less. Many other applications for the film exist beyond those few discussed herein.

### Film Former

The coatable composition of the present invention includes a polymeric film former that is water soluble or water dissolvable. Additionally, the film former is a thermoplastic material. For edible film products, the film former preferably meets the appropriate FDA regulations, and is tasteless and odorless.

The film former provides tensile strength to the resulting film product. As the molecular weight of the film former increases, so does the tensile strength. Additionally, as the molecular weight increases, the viscosity of the composition increases; lower molecular weight film formers are generally easier to compound with other ingredients. The flexibility of the resulting film product is dependent on the molecular structure of the film former rather than its molecular weight. Typically, if it is desired to modify the flexibility of the film product, a plasticizer (as discussed below) could be added.

Examples of suitable thermoplastic film formers for the present invention include hydroxypropyl cellulose (HPC), polyoxyethlene poly(2-ethyl-2-oxazoline) and similar polymers, polyvinylpyrrolidone (PVP), vinyl acetate, and copolymers and mixtures thereof. Other film formers that are suitable include carboxymethyl cellulose, ethyl cellulose, cellulose acetate phthalate, and pullulan. Still additional film formers that are suitable include polyoxyethylene (also known as Polyox), polymethyl vinyl ether/maleic anhydride copolymers (known as "Gantrez" from International Specialty Products), polyvinylpyrrolidone hydrogen peroxide complex (known as "Peroxydone" from International Specialty Products), polyvinyl alcohol (PVA), PVA/polyethylene glycol graft copolymer, polymethacrylates, polyacrylic acid, and polyacrylamide. Hydroxyl waxes can also be suitable as the film former in coatable compositions and in the coated film product of the present invention. Mixtures of these film formers can be used.

Some film formers may, together with the active material, include an amount of moisture or water, adsorbed or absorbed by the material. Additionally or alternately, some film formers may have chemically bound water to the material, as with the monohydrate and/or dihydrate forms. As an example, hydroxypropyl cellulose is about 6 wt-% water. This water or moisture may stay with the film former in the composition and the resulting film product, thus providing a water level to the final film.

The film former is present in the coatable composition at a level of at least 2 wt-%, and typically at least 5 wt-% based on the total weight of the composition. Usually, no more than 60 wt-% of the coatable composition is the film former, usually no more than 50 wt-%, typically no more than 45 wt-%, and often no more than 40 wt-% based on the total weight of the composition. A preferred range for the amount of film former in the coatable composition is 10 to 25 wt-% based on the total weight of the composition, and another preferred range is 20 to 45 wt-%.

Typically, the same amounts of film former are present in the resulting film as in the coatable composition. The film former is present in the film at a level of at least 2 wt-%, and typically at least 5 wt-% by total film weight. Usually, no more than 60 wt-% of the film is the film former, typically no more than 45 wt-% by total film weight. A preferred range for the amount of film former in the film is 10 to 25 wt-% by total film weight, and another preferred range is 20 to 45 wt-%, depending on the film former.

The preferred film former for an edible film product is hydroxypropyl cellulose, at a level of 15 wt-% of the coatable composition and 15 wt-% of the final film product.

When the amounts of components of the film are discussed herein, it is understood that the amounts of various components of the film can be ascertained in at least two ways. First, the components of the coatable composition that is coated to produce the film can be examined. Typically, the coated film and the film product will have the same components in the same amounts as the coatable composition. Second, the film itself can be chemically analyzed to determine its components.

In one embodiment, neither the edible film product nor the coatable composition includes more than 5 wt-% of other film formers, such as pullulan, starch, carboxymethyl cellulose, pectin or Acacia Senegal. In one embodiment, neither the edible film product nor the coatable composition includes more than 0.5 wt-% of these other film formers. In one embodiment, neither the edible film product nor the coatable composition includes any of these other film formers.

### Viscosity Modifier

The coatable composition of the present invention includes a viscosity modifier, which decreases the viscosity of the composition. Viscosity modifiers allow the composition to rapidly set to a non-tacky, solid film. Preferably, the viscosity modifier and has a sharp melting point and/or crystallization point.

The preferred viscosity modifier is a fatty alcohol. Fatty alcohols are generally water soluble, primary C8-C20 alcohols, usually straight chain. Preferred fatty alcohols to use are C8-C18. The alcohol can be a saturated or unsaturated alcohol. In one embodiment, the fatty alcohol is an unsaturated alcohol. The fatty alcohol is preferably a thermoplastic material. The fatty alcohol used can be either a solid or liquid at room temperature, however, those solid at room temperature are preferred in order to reduce the cooling and solidifying period of the coatable composition after coating. As stated above, a sharp melting point and/or crystallization point is desired for the fatty alcohol. Preferably, the melting point is at least 20 °C and no more than 120 °C. Additives such as soywax or other hydrogenated waxes, lecithin, stearin, palmintin and glycerol tristearate and glycerol triplaminitin could be added to adjust the melting point.

The film former is preferably soluble in the fatty alcohol.

Examples of suitable fatty alcohols include stearyl alcohol (also known as 1-octadecanol) which has a melting point of about 57-66 °C, myristyl alcohol (also know as 1-tetradecanol) which has a melting point of about 38 °C, and cetyl alcohol (also known as 1-hexadecanol) which has a melting point of about 52-54°C, although other fatty alcohols are also be suitable. Mixtures of these fatty alcohols can be used.

The fatty alcohol is included at a percentage weight level of the total film sufficient to cause the film to change from a solid to a liquid within a relatively narrow temperature range of 20 °C or less. The melting point of the fatty alcohol component influences the melting properties of the final film product. Where the fatty alcohol has a fairly sharp melting point, the film will more quickly disperse when in contact with body fluids such as saliva and the film will more quickly solidify and become non-tacky as it cools after extrusion. In addition, the fatty alcohol serves to lower the viscosity of a composition including a film former that is a high molecular weight polymer such as hydroxypropyl cellulose, thereby allowing the coatable composition to be more easily extruded.

Fatty alcohols such as stearyl alcohol cause the film to have a coating effect in the mouth, which is an advantage in some applications. For example, when the film is used for breath freshening, the coating action will cause the active ingredients to be well distributed in the mouth. When the film is used to deliver an analgesic or other medicament for a sore throat, the coating action will help evenly apply the medicament.

The fatty alcohol is present in the coatable composition at a level of at least 10 wt-%, and typically at least 15 wt-% based on the total weight of the composition. Usually, no more than 80 wt-% of the coatable composition is fatty alcohol, typically no more than 60 wt-% based on the total weight of the composition. A preferred range for the amount of fatty alcohol in the coatable composition is 20 to 50 wt-% based on the total weight of the composition. The fatty alcohol is present, as a ratio to the film former, at a level of 1:2 to 3:1 by weight in the coatable composition. Preferably, the ratio of fatty alcohol to film former is 2:1 by weight.

Similar levels of fatty alcohol are present in the film. The fatty alcohol is present in the film at a level of at least 10 wt-%, and typically at least 15 wt-%. Usually, no more than 80 wt-% of the film is the fatty alcohol, typically no more than 60 wt-%. A preferred range for the fatty alcohol in the film is 20 to 50 wt-%. The fatty alcohol is present, as a ratio to the film former, at a level of 1:2 to 3:1 by weight in the final film. Preferably, the ratio of fatty alcohol to film former is 2:1 by weight.

A preferred fatty alcohol for use with hydroxypropyl cellulose is stearyl alcohol. A preferred ratio of stearyl alcohol to hydroxypropyl cellulose is approximately 2:1 by weight.

Various other ingredients, such as polyisobutylene (such as "Oppanol" from BASF, and "Vistanex" from Exxon Mobile Chemical) could be used as viscosity modifiers. Waxes, including microcrystalline waxes (such as Multiwax from Crompton-Witco), hydrogenated castor oil or castor wax, soy wax, and hydroxyamide waxes may also be used.

It is noted that although these ingredients are classified herein as viscosity modifiers, they may also function as liquefiers or crystallizing agents,

In some embodiments it may be desired to increase the viscosity of the composition; that is, to thicken the composition. Although not preferred, a gelling agent could be used to thicken the composition. Examples of suitable thickeners or gelling agents include alginates, gums such as carrageenan hum, gellan gum, guar gum, xanthan gum, gelatin, acacia, cream of tarter, and pectin. Some ingredients discussed below under "Fillers or Bulking Agents" may also function as a thickening agent. Additionally, any of the ingredients provided above as viscosity modifiers may be used thicken the composition.

### Composition Modifiers

The composition may be further modified by various plasticizers, wetting agents, surfactants and/or emulsifiers. A plasticizer is generally known to increase the flexibility of the resulting film product by inhibiting crystallization of the film former by being distributed among the film former molecules, inhibiting crosslinking and crystallization. Wetting agents and surfactants are generally used to decrease the surface tension of the film product, which allows saliva and other liquids to better wet and penetrate the film. Wetting agents and surfactants generally increase the water solubility of the film former. An emulsifier may be used to improve the compatibility of various additive and adjuvants.

Examples of suitable modifiers that can be added to the composition and the film product include (in no particular order): ascorbic acid, polysorbate 20 (such as "Tween 20" from ICI Americas), polysorbate 80 (such as "Tween 80" from ICI Americas) and other polysorbates and sorbitans, "Span" emulsifiers from ICI Americas, ascorbyl palmitate, butanoic acid, propylene glycol, canola oil, coconut oil, peanut oil, cod liver oil, soybean oil, caster oil, sesame oil, olive oil, safflower oil, propylene glycol monocaprylate, citrate esters (such as "Citroflex NF" from Morflex), sorbitan monolaurate, "Sorbitol Special" solution from Pformulate, dibutyl sebacate, glycerin, stearic acid, glyceryl monolinoleate, glyceryl monooleate, glyceryl monostearate, glyceryl palmitostearate, triacetin, triethyl citrate, tributyl citrate, tristearin, glycerol triacetate, Vitamin E acetate, grape seed oil, linoleic acid, oleic acid, ethylene glycol, triethylene glycol, polyethylene glycol, polypropylene glycol, esters of propylene glycol with C4-C 12 fatty acids (such as "Capryol 90" from Gattefosse), palmitic acid, medium chain triglycerides (such as "Captex 200" from Abitec Corp.), lauric acid, benzyl benzoate, diethylene glycol monoethyl ester (such as "Transcutol"), sodium dodecyl sulfate, lauryl sulfate, lecithin, cyclodextrins, "Atmul" emulsifiers from Hercules, mono and diglycerides of fatty acids, acetylated monoglycerides (such as those known as "Myvacet" from Eastman Chemical Products), polyoxyethylene sorbitol esters, medium chain glycerides (such as those known as "Capmul" from Abitec Corp.), caprylocaproyl macrogolglycerides (such as those known as "Labrasol"), and poloxamers.

Addition of these ingredients is chosen based on their taste (or lack thereof), their compatibility with other ingredients of the composition, and other factors. Emulsifiers can be selected based on the HLB System, which is a standard system used for selecting emulsifiers used in the cosmetic industry.

### Fillers or Bulking Agents

The coatable composition of the present invention generally includes a filler or bulking agent to add solids and/or volume to the coatable composition and to the resulting film product. The filler or bulking agent provides a desired "mouth feel" to the film. Preferably, the filler or bulking agent selected dissolves or is soluble in the fatty alcohol and film former.

A carbohydrate is suitable as a filler. The filler can include a carbohydrate, such as a carbohydrate sugar having a molecular weight of at least 100 and at most 2000. Examples of suitable fillers or bulking agents include dextrose, sorbitol, polydextrose, trehalose, maltose, maltodextrin, corn syrup solids, mannitol, xylitol, cyclodextrins, calcium carbonate, starches (including corn starch and rice starch) and mixtures thereof. Gelatin and cellulose may be suitable filler or bulking agents. It is understood that for edible films of the invention, other food grade fillers or bulking agents could be used. For films intended for topical application, it is not necessary that the fillers or bulking agents are food grade.

The bulking agent is preferably mostly tasteless or has a sweet taste and preferably does not have a strong bad taste. The bulking agent can also serve a role as a sweetener, as is the case with dextrose, sorbitol, mannitol, xylitol, cyclodextrins and mixtures thereof. By taking up space in the composition, the bulking agent overcomes the somewhat slimy mouth feel of a viscous film former such as hydroxypropyl cellulose. Preferably the bulking agent does not react with any of the other ingredients of the coatable composition.

The bulking agent will likely have a lower molecular weight than the film former. The molecular weight of dextrose (glucose) is about 180, which includes one glucose ring. The disaccharides, like maltose, lactose, and sucrose, include two glucose rings and have a molecular weight about twice that of dextrose, at about 342. For polydextrose substances with multiple glucose rings the molecular weight could be up to 1000 or 2000. In one embodiment, the bulking agent has a molecular weight of at least 150 and not more than 2000. In another embodiment, the bulking agent has a molecular weight of at least 100 and not more than 600.

In one embodiment, dextrose monohydrate is included in the coatable composition as a bulking agent. Dextrose monohydrate typically includes about 6-8 wt-% water by weight. Dextrose monohydrate melts at about 185 °F (85 °C) and will not degrade or char until it reaches a temperature of about 265 °F (129 °C) or higher. Anhydrous dextrose can also be used as a bulking agent. In one embodiment, the bulking agent is a material that does not degrade or char when it melts, and preferably, does not degrade or char at a temperature within 10 °C of its melting point. Preferably, the bulking agent does not degrade or char at temperatures of 275 °F (135 °C) or lower. In another embodiment, the bulking agent is a material that does not degrade or char at temperatures of 265 °F (129°C) or lower.

U.S. Patent 6,596,298 describes an oral care film including a water soluble film-forming polymer and essential oils and emphasizes that oral care films having a high oil content should avoid substantial amounts of humectant in the film, and preferably have no humectant, to avoid producing an overly moist, self-adhering film. However, the coated film of the present invention does not become overly moist or self-adhering when a humectant is included, even when essential oils are included. In one embodiment, the coated film includes up to 40-50 wt-% of dextrose, which is a humectant, without adverse effects. The mixture of hydroxypropyl cellulose, fatty alcohol and dextrose results in a film with desirable characteristics, that is not self-adhering, even when oils are included.

The filler or bulking agent is present in the coatable composition at a level of at least 20 wt-%, and typically at least 25 wt-% based on the total weight of the composition. Usually, no more than 75 wt-% of the coatable composition is the filler or bulking agent, typically no more than 70 wt-%. A preferred range for the amount of filler or bulking agent in the coatable composition is 40 to 60 wt-%.

Typically, the same levels of bulking agent are present in the film as are present in the coatable composition. The bulking agent is present in the film at a level of at least 20 wt-%, and typically at least 25 wt-% by total film weight. Usually, no more than 75 wt-% of the film is the filler or bulking agent. A preferred range for the amount of filler or bulking agent in the film is 40 to 60 wt-% by total film weight.

### Sweetener

If the film product is to be orally administered, it is preferred that the coating composition, and the resulting film product, includes a sweetener. Also, if the film product includes a medicament with bitter or undesirable taste, the sweetener can have a masking effect and hide the taste of the medicament. The sweetener can be a natural sweetener or an artificial sweetener or include both.

Examples of natural sweeteners include monosaccharides such as glucose (dextrose), fructose (levulose), galactose, dextrose derivatives, dextrin, dextrin derivatives such as maltodextrin, disaccharides such as sucrose, maltose, lactose and cellobiose, and mixtures of these.

Additional examples of natural sweeteners include polysaccharides such as maltodextrin, starch, amylase and amyl pectin. Further examples of natural sweeteners include xylose, ribose, mannose, invert sugar (a mixture of fructose and glucose derived from sucrose), partially hydrolyzed starch, corn syrup solids, dihydrochalcones, monellin, steviosides, and glycyrrhizin. Crystalline hydrate forming sugars, which include maltose, lactose, and also isomalt, trehalose, and raffinose, can also be suitable natural sweeteners to use. Mixtures of these sweeteners can also be used.

Many different natural and artificial sweeteners can be used. For example, U.S. Patent 6,596,298 describes an oral care film and discusses many different sweeteners that can be included in a film. The film of the present invention can include any of the sweeteners that are described and listed in U.S. Patent 6,596,298.

It is realized that various ingredients can act as both a sweetener and as a filler or bulking agent, ingredients such as dextrose, sorbitol, mannitol, xylitol, cyclodextrins and mixtures of these.

Since a typical breath freshener strip, as illustrated in FIGS.1 and 2 weighs only approximately 0.3 grams, it is difficult to incorporate a sufficient amount of natural sweetener to give the desired "burst" of sweetness. Thus preferably, the sweetener used for the coating composition includes an artificial sweetener, which are significantly more sweet than sugar. For example, saccharin is about 300 times sweeter than sugar, acesulfame K is about 200 times as sweet as sugar, sucralose has about 450 to 650 times the sweetness of sugar, and aspartame is about 200 times as sweet as sugar.

The sweetener is present in the coatable composition at a level of at least 0.5 wt%, and typically at least 1 wt-% based on the total weight of the composition. Usually, no more than 25 wt-% of the coatable composition is the sweetener, typically no more than 20 wt-% based on the total weight of the composition. A preferred range for the amount of sweetener in the coatable composition is 2 to 10 wt-% based on the total weight of the composition.

Typically, similar levels of sweetener are present in the coated film. The sweetener is present in the film at a level of at least 0.5 wt-%, and typically at least 1 wt-% by total film weight. Usually, no more than 25 wt-% of the film is the sweetener. A preferred range for the amount of sweetener in the film is 2 to 10 wt-% by total film weight.

### Combined Sweetener and Bulking agent

To facilitate processing, the sweetener and bulking agent can be obtained combined, and used in such form. Examples of suitable materials which are a combination of a sweetener and a bulking agent or filler include SWEETONE® sugar substitute from Stadt Corp., which includes approximately 85-98 wt-% dextrose and 2-15 wt-% acesulfame K, and SPLENDA® sweetener from McNeil Specialty Products Company, which is sucralose with a maltodextrin carrier. A combined material of aspartame and maltodextrin is also available. Mixtures of these materials can also be used. Although the carriers, such as dextrose and maltodextrin, can be a sweetener in their own right, it is understood that the sweetening effect from products such as SWEETONE®) sugar substitute and SPLENDA® sweetener results mainly from the artificial sweetener component.

The combination of sweetener/bulking agent is present in the coatable composition at a level of at least 20 wt-%, and typically at least 35 wt-% based on the total weight of the composition. Usually, no more than 75 wt-% of the coatable composition is the sweetener/bulking agent, typically no more than 60 wt-% based on the total weight of the composition. A preferred range for the sweetener/bulking agent in the coatable composition is 45 to 55 wt-% based on the total weight of the composition.

Similarly, the sweetener/bulking agent is present in the film at a level of at least 20 wt-%, and typically at least 35 wt-% by total film weight. Usually, no more than 75 wt-% of the film is sweetener/bulking agent and typically no more than 60 wt-% by total film weight. A preferred range for the sweetener/bulking agent in the film is 45 to 55 wt-% by total film weight.

### Adjuvants

The film product will typically include other ingredients selected based on the resulting use of the film product. For example, edible films can include a flavorant, such as peppermint, menthol, or other suitable flavor for the film. Medicinal films, whether edible or for topical application, will include a medicament. Nutrients such as vitamins and minerals can be included in edible films

Adjuvants such as flavorants, medicaments, nutrients and other additives such as color (dyes, pigments), can be added to the coatable composition so that the resulting film includes the additive. Many flavorants, medicaments, nutrients and dyes are water-based products; also, oil based products could be used. It is preferred that any additives are used in levels to not appreciably disrupt the thermoplastic properties of the coatable composition.

Examples of suitable colorants, dyes or pigments that could be used include FD&C Red #40, FD&C Blue #1, FD&C Blue #2, FD&C Yellow #5, tartrazine and annatto oil. Particulates such as titanium dioxide, aluminum oxide, and zinc oxide can also be used to impart color. It is noted that, preferably, particulates are micronized or otherwise finely ground to submicron sizes prior to incorporation into the composition.

By use of the term "flavorant", what is intended is an ingredient that imparts a desired flavor and/or odor to the film product. Examples of usable flavorants include natural oils and essential oils. For example, peppermint oil, menthol, eucalyptol, cinnamon flavoring, cherry flavoring, lemon flavoring or oil, grape flavoring, clove oil and other oils, vanillin, cacao, fruit extracts, honey, mixtures thereof, and the like can be used. Artificial flavors can also be used.

It is understood that generally any flavorant can be used, including those described in "Chemicals Used in Food Processing", publication 1274 by the National Academy of Sciences. For example, U.S. Patent 6,596,298 describes an oral care film and discusses many different flavorants that can be included in a film. The film of the present invention can include any of the flavorants that are described and listed in U.S. Patent 6,596,298, which was previously incorporated herein.

Another type of adjuvant that can be present in a film intended for oral use is a masking agent, designed to mask an undesirable taste that sweeteners may not hide or cover. Suitable taste masking agents include "Cremophor" from BASF, adenosine monophosphate, monosodium glutonate, "Amberlite IRP-69" and "Amberlite IRP-88" ion exchange resin from Quantyka, and sulfonated polystyrene crosslinked with divinylbenzine ion exchange resins, such as "Dow XYS-40010.00" from Dow Chemical. A sweetener can be used to mask other flavors, or other masking agents can be included. An example of a taste masking sweetener is monoammonium glycyrrhizinate, available as "MagnaSweet" from Mafco.

Essential oils are generally compatible with the film former hydroxypropyl cellulose and fatty alcohol, so that a coatable composition including these three elements can be mixed together easily. As a result, it is typically not necessary to utilize an emulsification process to combine the essential oil, component hydroxypropyl cellulose and fatty alcohol.

The amount of adjuvants, in the coatable composition is typically no more than 20 wt-% of the coatable composition, typically no more than 10 wt-% by total weight of the composition. A preferred range for the amount of such additives in the coatable composition is 0.5 to 5 wt-%, more preferred 1 to 2 wt-% by total weight of the composition. However, for some applications a medicament is as much as 40 wt-% by the total weight of the coatable composition.

Similar amounts of these ingredients are found in the film. The amount of flavorants, colorants, medicaments, if present, in the final film product is no more than 20 wt-%, typically no more than 10 wt-% by total film weight. A preferred range is 0.5 to 5 wt-%, more preferred 1 to 2 wt-% by total film weight. For some applications, a medicament is as much as 40 wt-% of the film by total film weight.

Alternately, any adjuvants can be coated onto the film product after production of the film. An additive can be sprayed onto the film after it is coated, either before the film has completely cooled or after it has cooled to the ambient temperature. A coating on the film product can be useful to mask the taste of the active ingredient or to prevent the active ingredient from numbing the tongue or other surfaces in the oral cavity. The coatings that can be used for this purpose are known to those skilled in the art, including polymers, celluloses, such as ethylcellulose, mixtures thereof and the like.

It can be desirable to print something on the surface of the film for many applications. Printing techniques for printing on flexible films are known in the art and could be applied to this film. For films intended for oral consumption, a food-grade ink will be used.

By use of the term "medicament" and variations thereof, what is intended is an ingredient that provides a therapeutic effect. An example of a medicament, for sore throat relief, is benzocaine. For topical applications, aloe or other skin care agents can be included.

Examples of medicaments that can be included in the film include:
A. antimicrobial or antibacterial agents, such as triclosan, cetyl pyridium chloride, domiphen bromide, quaternary ammonium salts such as benzalkonium chloride, zinc compounds, sanguinarine, fluorides, alexidine, octonidine, EDTA, benzyl alcohol, benzoic acid, benzyl chloride, benzethonium chloride, hydroxybenzoates (which are a type of paraben), sodium azide, mixtures thereof and the like,
B. non-steroidal anti-inflammatory drugs, such as aspirin, acetaminophen, ibuprofen, ketoprofen, diflunisal, fenoprofen calcium, naproxen, tolmetin sodium, indomethacin, mixtures thereof and the like,
C. anti-tussives, such as benzonatate, caramiphen edisylate, menthol, dextromethorphan hydrobromide, chlophedianol hydrochloride, mixtures thereof and the like,
D. decongestants, such as pseudoephedrine hydrochloride, phenylepherine, phenylpropanolamine, pseudoephedrine sulfate, mixtures thereof and the like,
E. anti-histamines, such as brompheniramine maleate, chlorpheniramine maleate, carbinoxamine maleate, clemastine fumarate, dexchlorpheniramine maleate, diphenhydramine hydrochloride, diphenylpyraline hydrochloride, azatadine meleate, diphenhydramine citrate, doxylamine succinate, promethazine hydrochloride, pyrilamine maleate, tripelennamine citrate, triprolidine hydrochloride, acrivastine, loratadine, brompheniramine, dexbrompheniramine, mixtures thereof and the like,
F. expectorants, such as guaifenesin, ipecac, potassium iodide, terpin hydrate, mixtures thereof and the like,
G. anti-gas additives, such as dimethicone, simethicone, aluminum hydroxide, and the like, and
H. caffeine.

Many different medicaments can be included in the film. U. S. Patent 6,596,298 lists many different examples of pharmaceutical agents that can be included in a film. The film of the present invention can include any of the pharmaceutical agents, dose amounts and other substances that are described and listed in U. S. Patent 6,596,298.

Various other optional adjuvants may be present in the composition and the film product.

For oral films, a saliva stimulant may be added to increase salivation of the user. Examples of saliva stimulants include citric acid, malic acid, and ascorbic acid, although others are known and could be used.

Additionally or alternately, a disintegrant, which absorbs water, such swelling and fracturing the film product, could be used. Examples of disintegrants include crospovidone, croscannellose, microcrystalline cellulose, sodium starch glycolate, croscannellose sodium, gellan gum and starch DC.

A foaming agent could also be added to the composition to modify the resulting film product; a foaming agent would function in a manner similar to a disintegrant, facilitating the fracturing of the film product. Examples of useable foaming agents include casseinate and whey.

It may be desired to add an antioxidant to the composition, for example, to increase the shelf life of the film product. Examples of antioxidants include butylated hydroxytoluene (BHT), butylated hydroxyanisole (BHA), citric acid, ascorbic acid, erythoribic acid, "Ionox 100" from Chance & Hunt, sodium metabisulfite, gallic acid ester, tartaric acid, lecithin, glycine, tocopherols, tertiary butyl hydroquinon, and Vitamin E TPGS (such as available from Eastman Chemical Co.).

Examples of other adjuvants that may be added to the composition and the film product include (in no particular order): calcium stearate, magnesium stearate, diethyl phthalate, diethyl sebecate, sodium lauryl sulfate, sodium laureth sulfate, cysteine, sodium phosphate monobasic, sodium phosphate dibasic, sodium phosphate tribasic, tea tree oil, calcium phosphate dibasic, calcium phosphate tribasic, and calcium silicate.

Some of the ingredients discussed above may include an amount of moisture or water, adsorbed or absorbed by the material. Additionally or alternately, some ingredients may have chemically bound water to the material, such as with the monohydrate and/or dihydrate forms. As an example, dextrose monohydrate includes about 6-8 wt-% water. This water or moisture may stay with the ingredient in the composition and the resulting film product, thus providing a water level to the final film.

### Preferred Compositions

One preferred coatable composition has the following components where amounts are specified based on the total weight of the composition: 30 wt-% fatty alcohol (i.e., stearyl alcohol), 53.2 wt-% sweetener and filler (i.e., SWEET ONE® sugar substitute) which provides about 4 wt-% acesulfame K and about 49.2 wt-% dextrose, 15 wt-% film former (i.e., hydroxypropyl cellulose), and 1.8 wt-% flavorant (i.e., 0.8 wt-% peppermint oil and 1.0 wt-% menthol). There is no water added to this composition.

Another preferred coatable composition has the following components where amounts are specified based on the total weight of the composition: 35 wt-% fatty alcohol (e.g., stearyl alcohol), 49 wt-% sweetener and filler (i.e., SWEET ONE® sugar substitute) which provides about 3.7 wt-% acesulfame K and about 45.3 wt-% dextrose, 15 wt-% film former (e.g., hydroxypropyl cellulose), and 1 wt-% flavorant (e.g., peppermint oil). No water is added to this composition. Any water present in the composition is from the 6-8% moisture content of the hydroxypropyl cellulose and dextrose. This water is partially evaporated during the mixing steps. In one embodiment, this water is removed from the coatable composition by applying a vacuum during the mixing steps.

The low water content of the film is advantageous. Preferably the water content of the film is 10 wt-% or less, and can be 8 wt-% or less or preferably 6 wt-% or less by total film weight. The water content of the film can be 5 wt-% or less, 2 wt-% or less, 0.5 wt-% or less by total film weight, or the film can lack any detectable water content.

### Mixing the Coatable Composition

The coating composition is prepared by combining the desired ingredients in any suitable manner to form a mixture. It is preferred that the resulting coatable composition is thoroughly mixed with a smooth consistency. The ingredients in the coatable composition can be completely solvated, or, the ingredient can be mixed together retaining their individual states. The coatable composition can be homogeneous, although this is not necessary and the coatable composition is not homogenous in one embodiment.

A preferred method for preparing the coatable composition is to mix the film former into the fatty alcohol, which is in a liquid form. If solid, the fatty alcohol is melted or otherwise liquefied prior to adding the film former. The fatty alcohol provides a solvent for dispersing or melting the film former therein. As stated above, preferably the film former dissolves or at least is soluble in the fatty alcohol.

Optional ingredients, such as sweetener and/or bulking agent, can be added to the fatty alcohol either before or after the film former is added. The particular order of addition will depend on the amount and types of ingredients used. In the preferred method, water is not added to the coatable composition during mixing in any appreciable quantity. Although water can be a part of some of the ingredients, such as hydroxypropyl cellulose and dextrose, it is not introduced by itself in quantities of greater than 2 wt-%, and preferably water alone is not introduced at all.

After all ingredients are mixed and prior to extrusion, the coatable composition preferably has a viscosity of less than 150,000 centipoise at 121 °C, more preferably 125,000 centipoise.

### Extruding the Coatable Composition

The coating composition, as a liquid, is coated to provide a coated film that can be cut into the resulting film product. Examples of suitable coating methods include extrusion coating, knife coating, roll coating, and the like, however due to the thermoplastic nature of the coatable composition, the preferred coating method is by extrusion at elevated temperatures.

Typically, the coatable composition is extruded through a slot die to form the film product. The coatable composition can be provided to the extruder as a liquid and then extruded, or, the coatable composition can be provided to the extruder as a solid material, which is then melted by the extruder or other equipment upstream of the extruder. Combining of the ingredients can be done prior to providing to the extruder or can be done by the extruder. For embodiments where the individual ingredients are provided to the extruder and then mixed *is situ,* a twin-screw extruder is the preferred equipment. In some applications, combining the ingredients in the extruder is preferred, as the evaporation of moisture or other volatile substances such as flavorants from the composition can be inhibited.

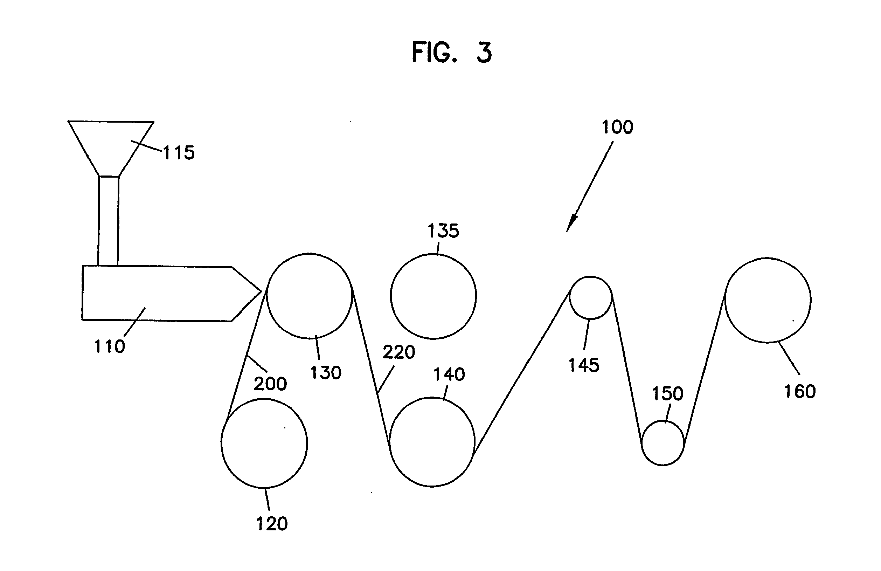

FIG. 3 is a schematic depiction of an exemplary process for coating the coatable composition of the present invention. Coating system 100 is a typical extrusion coating system; those skilled in the art of coating will understand that numerous variations of system 100 could be used for providing the film product of the present invention.

Referring to FIG. 3, system 100 includes an extruder 110 for providing liquid coatable composition. In the system illustrated as 100, the coatable composition is fed to extruder 110 via hopper 115, which retains and feeds solid particulate of the coatable composition to extruder 110. Extruder 110 melts the solid coatable composition to form a coatable liquid. A preferred extruder 110 for such a system is a single screw extruder.

In an alternate coating system, the coatable composition could be fed to extruder 110 in a liquid state. For such a set-up, it can be desirable to feed the coatable composition to extruder 110 via heated pipes or tubes, to inhibit the composition from setting up prior to reaching extruder 110. In yet another alternate coating system, the ingredients for the coatable composition, such as the film former and the fatty alcohol, could be fed individually to extruder 110. Extruder 110 would compound and melt the ingredients; a preferred extruder for such a system would be a twin screw extruder.

The preferred coating method for the thermoplastic coatable composition of the present invention is with a slot die. Slot dies are well known in the art of coating, and include a manifold for receiving liquid material and a slot for expelling the material. Extruder 110 provides a steady supply of liquid material to the manifold to provide a consistent coating through the slot by applying pressure greater than atmospheric temperatures to the liquid material. The liquid material is at a temperature above the melting point of the coating composition; typically, the liquid material is at least 150 °F (66 °C). For a composition including stearyl alcohol, an extrusion temperature of at least 200 °F (93 °C), more preferably at least 225 °F (107 °C) is desired. The liquid material is preferably not heated above 250 °F (122 °C). To provide a final film thickness of 2 mils, the height of the slot is 2 mils or 0.05 mm. Similarly, to provide a film 5 mils or 0.1 mm thick, the height of the slot is 5 mils or 0.1 mm.

Referring again to FIG. 3, system 100 includes a roll 120 of carrier web 200. The coatable composition is extruded onto carrier web 200 by extruder 110. Typically, a back-up roll or contact roll 130 is used; often, contact roll 130 is a driven roll. Coated web 220, which has a coating of coatable composition on carrier web 200, is fed to idler roll 140. A nip roll 135 can be included to facilitate movement of web 220. Nip roll 135 can be any suitable roll, for example, a steel roll or a rubber roll. Roll 135 can be chilled to speed the cooling and solidification of the coatable composition. However, with some coatable compositions, the cooling of the extruded material due to the drop from the extrusion temperature (e.g., 225-250 °F, 107-121 °C) to ambient temperature, the extruded film will already have cooled and solidified prior to reaching roll 135.

After extrusion at an elevated temperature, the coatable composition quickly cools to a non-tacky state. It is not necessary to dry the film to drive off moisture or solvents. It is not necessary to apply heat to the film and circulate air for the extruded film to become non-tacky. By "non-tacky" it is meant that the film does not have an appreciable tack or stickiness upon touching it at typical ambient temperatures of 21-23 °C. The non-tacky film does not significantly deform and adhere to other surfaces at temperatures of 21-23 °C. Preferably the film is also non-blocking, so that it does not stick to itself. As a result, the film can be wound without a top liner in one embodiment. Because the film becomes non-tacky simply by cooling, and drying steps are not necessary, the film can be extruded at faster line speeds compared to films that require drying or driving off solvents. Also, the coated film can be easily separated from the carrier web substrate.

System 100 includes various other rolls, such as rolls 145,150. Such rolls are known to control the tension of web 220. One skilled in the art of coating will be able to design an appropriate set up. Web 220 is collected as roll 160.

The resulting coated film product has a thickness of 1 mil to 5 mil (0.03 to 0.1 mm), For an edible film product, a thickness of 2 mil (0.05 mm) is preferred.

Run speeds for system 100 are generally at least 10 feet per minute and typically at least 50 feet per minute. Speeds of 100 feet per minute and more would be typical for commercial coating processes. The nature of the coatable composition, being a thermoplastic, allows the extruded film to solidify within 10 seconds of coating. Depending on the specific composition, solidification can occur within 5 seconds of coating, and even within 1 second of coating. Coating compositions having lower amounts of water, e.g., no more than 5 wt-% by total composition weight, will dry or solidify quicker than compositions having higher amounts of water. Once solidified, the film product is non-tacky to the touch. As the film further crystallizes, the film becomes more rigid and handleable.

The resulting web of film can be slit using conventional techniques. Due to the thermoplastic properties of the film, excess material, such as the head and tail of the web and the side edges, can be removed from the usable film and recycled to the extruder. That is, the film can be remelted and coated again.

### Examples

The following examples are not limiting, but are provided for a better understanding of the invention. All percentages and parts are provided as weights, based on the weight of the coatable composition unless otherwise noted.

### Example 1

First, 600 grams of stearyl alcohol (commercially available from Croda under the designation "Crodacol S-95 NF") was melted in a Sigma high shear mixer at a temperature of 225 °F (107.2 °F). To this melted fatty alcohol were added 500 grams of a mixture of dextrose and acesulfame K (commercially available from Stadt Corp. under the designation"Sweet One"), The mixture was stirred and heated at 200-225 °F (93.3-107.2 °C) until the dextrose melted. Acesulfame was present dispersed in the melted mixture of dextrose and fatty alcohol. To this, 300 grams of hydroxypropyl cellulose (commercially available from Hercules under the designation KLUCEL® EF polymer) were added and mixed at 225 °F (107.2 °C) until thickened. An additional 564 grams of SWEETONE® sugar substitute were added and mixed at about 225 °F (107.2 °C). The mixture appeared a little grainy. Next, 16 grains of peppermint oil and 20 grams of menthol were added to the mix; the resulting mix was smoother with the oils added. The coatable composition was fairly thick, with a viscosity of about 125,000 centipoise at 121 °C.

The coatable composition had 30 wt-% fatty alcohol (i.e., stearyl alcohol), 4 wt-% sweetener (i.e, acesulfame K), 49.2 wt-% filler (i,e., dextrose), 15 wt-% film former (i.e., hydroxypropyl cellulose), and 1.8 wt-% flavorant based on the total weight of the composition. No water was added to the coatable composition.

The coatable composition was pulled with a metal ring to a thickness of 0.05 mm (2 mil) onto silicone release paper. Within 1 second of being coated, the film composition had hardened and was non-tacky to the touch. After 10 seconds, the film was cut to about 2.5 cm by 5.1 cm (1 inch by 2 inch) and removed from the wax paper. The film was sufficiently rigid and self-supporting to withstand handling.

The film was tasted and had a peppermint flavor. The film dissolved on the tongue within 2 seconds and left no undesirable aftertaste or sticky or pasty feeling.

### Example 2

Example 2 was prepared similar to Example 1, except that 564 grams of the dextrose/acesulfame K were added to the melted stearyl alcohol rather than 500 grams, This mixture required longer heated mixing than Example 1. The remaining 500 grams of the dextrose/acesulfame K were added after the 300 grams of hydroxypropyl cellulose.

The coatable composition had 30 wt-% fatty alcohol (i.e., stearyl alcohol), 4 wt-% sweetener (i.e., acesulfame K), 49.2 wt-% filler (i.e., dextrose), 15 wt-% film former (i.e., hydroxypropyl cellulose), and 1.8 wt-% flavorant based on the total weight of the coatable composition.

Although the resulting coatable composition looked the same as that from Example 1, this coatable composition was not coated to form a film.

### Example 3

First, 700 grams of stearyl alcohol were melted. To this melted fatty alcohol were added 980 grams of SWEET ONE® sugar substitute. To this, 300 grams of hydroxypropyl cellulose were added. Next, about 2 grams of peppermint oil were added to the mix.

The coatable composition had 35 wt-% fatty alcohol (i.e., stearyl alcohol), 3.7 wt-% sweetener (i.e., acesulfame K), 45.3 wt-% filler (i.e., dextrose), 15 wt-% film former (i.e., hydroxypropyl cellulose), and 1 wt-% flavorant, where the quantities are based on the total weight of the composition. No water was added to this composition.

The coatable composition was coated as described in Example 1.

### Example 4

First, 600 grams of stearyl alcohol were melted at a temperature of 225 °F. To this, 300 grams of hydroxypropyl cellulose were added and mixed at 225 °F until thickened, about 20 minutes. To this was added 246 grams of dextrose monohydrate. The mixture was stirred and heated at 225 °F until the dextrose melted, about 10 minutes. Then, 200 grams of sorbitol was added; when fully incorporated, 4 grams of sucralose and 4 grams of Acesulfame K (fine grind) were added. An additional 600 grams of dextrose monohydrate were added and mixed at 225 °F until thickened, about 20 minutes. Next, 24 grams of peppermint oil and 16 grams of menthol were added and the mixture was mixed for 5 minutes at 225 °F.

The coatable composition had 30 wt-% fatty alcohol (i.e., stearyl alcohol), 0.4 wt-% sweetener (i.e., sucralose and acesulfame K), 52.3 wt-% filler (i.e., dextrose and sorbitol), 15 wt-% film former (i.e., hydroxypropyl cellulose), and 2.3 wt-% flavorant based on the total weight of the composition. No water was added to the coatable composition.

### Example 5

First, 600 grams of stearyl alcohol together with 184 grams dextromethorphan were melted at a temperature of 225 °F in the Sigma mixer. To this, 432 grams of hydroxypropyl cellulose were added. The mixer temperature was increased to 265 °F and the mixture was mixed about 30 minutes. 4 grams sucralose and 16 grams of sorbitol were mixed and ground to break up any lumps. This sweetener pre-mix was to the melted stearyl alcohol and HPC, after which 40 grams of polysorbate 80 were added. Separately, 93 grams of sorbitol and 1 gram of FD&C Red #40 were mixed and melted at 300 °F. This colored pre-mix and 280 additional grams of sorbitol were added to the melted stearyl alcohol and HPC and mixed at 265 °F for 10 minutes. Then, 344 grams of microcrystalline cellulose (type 105) were added. The temperature was decreased to 225 °F and the mixture was mixed for 15 minutes. To this was added 6 grams of natural cherry flavor. After 5 more minutes of mixing, the composition was removed from the mixer.

The coatable composition had 30 wt-% fatty alcohol (i.e., stearyl alcohol), 0.2 wt-% sweetener (i.e., sucralose), 38.65 wt-% filler (i.e., sorbitol, polysorbate 80 and microcrystalline cellulose), 21.6 wt-% film former (i.e., hydroxypropyl cellulose), 9.2 wt-% active (i.e., dextromethorphan), 0.05 wt-% colorant, and 0.3 wt-% flavorant based on the total weight of the composition. No water was added to the coatable composition.

### Example 6

The ingredients for this example were the same as used for Example 5. These ingredients, however, were all placed into and mixed in a twin-screw extruder.

Various modifications and alterations of this invention will become apparent to those skilled in the art.

It is to be understood that even though numerous characteristics and advantages of the present disclosure have been set forth in the foregoing description, together with details of the coatable compositions and the film products, the disclosure is illustrative only, and changes can be made in detail, especially in matters of shape, size and arrangement of parts and types of materials within the principles of the disclosure to the full extent indicated by the broad general meaning of the terms in which the appended claims are expressed.

## Claims

1. A film formed by the method including the steps of:
(a) providing a water-soluble film former;
(b) thoroughly mixing the film former in a fatty alcohol component to form a mixture, the fatty alcohol component present at a percentage weight level of the total film sufficient to cause the film to change from a solid to a liquid within a relatively narrow temperature range of 20 °C or less; and
(c) extruding the mixture to form a coated film, wherein the mixture is at a temperature of at least 65 °C when extruded;
(d) so that when the coated film cools to 30 °C or lower, the extruded film is non-tacky.

2. The film according to claim 1, wherein:
(a) the film former includes liquid stearyl alcohol.

3. The film according to claim 1, wherein:
(a) the water soluble film former is thermoplastic.

4. The film according to claim 1, wherein:
(a) the water soluble film former is hydroxypropyl cellulose.

5. The film according to any of claims 1-4, wherein:
(a) the mixture is extruded through a slot die.

6. A method of making a film, the method including the steps of:
(a) providing a water-soluble film former;
(b) thoroughly mixing the film former in a fatty alcohol component to form a mixture, the fatty alcohol component present at a percentage weight level of the total film sufficient to cause the film to change from a solid to a liquid within a relatively narrow temperature range of 20 °C or less; and
(c) extruding the mixture to form a coated film, wherein the mixture is at a temperature of at least 65°C when extruded.

7. A method of making a film, the method including the steps of:
(a) providing a water-soluble film former;
(b) thoroughly mixing the film former with a fatty alcohol to form a mixture having less than 5 wt. % water, wherein the mixture comprises fatty alcohol at a percentage weight level of the total film sufficient to cause the film to change from a solid to a liquid within a relatively narrow temperature range of 20°C or less; and
(c) extruding the mixture to form a coated film, wherein the mixture is at a temperature of at least 65 °C when extruded.

8. The method according to any of claims 6-7:
(a) wherein the fatty alcohol component melts at a temperature of at least 20°C and less than 120 °C.

9. The method according to any of claims 6-8:
(a) wherein the fatty alcohol component is included at a percentage weight level of the total film sufficient to cause the mixture to have a viscosity of 150,000 centipoise or less.

10. The method according to any of claims 6-9:
(a) wherein the fatty alcohol component includes a straight-chain C8-C20 alcohol.

11. The method according to any of claims 6-10:
(a) wherein the fatty alcohol component includes at least one selected from the group consisting of myristyl alcohol, cetyl alcohol, stearyl alcohol and mixtures thereof.

12. The method according to any of claims 6-11, wherein the step of mixing the film former comprises:
(a) mixing the film former in liquid stearyl alcohol.

13. The method according to any of claims 6-12, wherein:
(a) the water soluble film former is thermoplastic.

14. The method according to any of claims 6-13, wherein the step of providing a water soluble film former comprises:
(a) providing hydroxypropyl cellulose,

15. The method according to any of claims 6-14, wherein the step of extruding the mixture comprises:
(a) extruding the mixture through a slot die.

16. The method according to any of claims 6-14, further including the steps of:
(a) prior to extruding the mixture to form the coated film, solidifying the mixture to form a solid material;
(b) melting the solid material to form a melted material; and
(c) extruding the melted material.

17. The method according to any of claims 6-16, further including the steps of, after extruding the mixture:
(a) trimming away a portion of the coated film at edges of the coated film;
(b) melting the trimmed-away portion of the coated film to form a melted material; and then
(c) extruding the melted material to form a second coated film.

18. The method according to any of claims 6-17, where the step of mixing the film former with a substance to form a mixture comprises:
(a) mixing the film former with a substance to form a mixture having less than 2 wt-% water.

19. The method according to any of claims 6-18, where the step of mixing the film former with a substance to form a mixture comprises:
(a) mixing the film former with a substance to form a mixture having no added water.

20. The film according to claim 1, wherein the fatty alcohol component is at least 10 wt-% by total film weight.

21. The method according to any of claims 6-7, wherein the fatty alcohol component is at least 10 wt-% by total film weight.

## Patentansprüche

1. Film, der durch das Verfahren gebildet wird, das die Schritte:
(a) Bereitstellen eines wasserlöslichen Filmbildners;
(b) gründliches Einmischen des Filmbildners in eine Fettalkoholkomponente unter Bildung eines Gemisches, wobei die Fettalkoholkomponente in einer Prozentgewichtskonzentration des Gesamtfilms vorliegt, die ausreichend ist, um zu bewirken, dass der Film in einem relativ engen Temperaturbereich von 20°C oder weniger von einem Feststoff in eine Flüssigkeit übergeht; und
(c) Extrudieren des Gemisches unter Bildung eines aufgetragenen Films, wobei das Gemisch eine Temperatur von wenigstens 65°C hat, wenn es extrudiert wird;
(d) so dass der extrudierte Film, wenn der aufgetragene Film auf 30°C oder weniger abkühlt, nicht klebrig ist,
umfasst.

2. Film gemäß Anspruch 1, wobei:
(a) der Filmbildner flüssigen Stearylalkohol umfasst.

3. Film gemäß Anspruch 1, wobei:
(a) der wasserlösliche Filmbildner thermoplastisch ist.

4. Film gemäß Anspruch 1, wobei:
(a) der wasserlösliche Filmbildner Hydroxypropylcellulose ist.

5. Film gemäß einem der Ansprüche 1 bis 4, wobei:
(a) das Gemisch durch eine Breitschlitzdrüse extrudiert wird.

6. Verfahren zur Herstellung eines Films, wobei das Verfahren die folgenden Schritte umfasst:
(a) Bereitstellen eines wasserlöslichen Filmbildners;
(b) gründliches Einmischen des Filmbildners in eine Fettalkoholkomponente unter Bildung eines Gemisches, wobei die Fettalkoholkomponente in einer Prozentgewichtskonzentration des Gesamtfilms vorliegt, die ausreichend ist, um zu bewirken, dass der Film in einem relativ engen Temperaturbereich von 20°C oder weniger von einem Feststoff in eine Flüssigkeit übergeht; und
(c) Extrudieren des Gemisches unter Bildung eines aufgetragenen Films, wobei das Gemisch eine Temperatur von wenigstens 65°C hat, wenn es extrudiert wird.

7. Verfahren zur Herstellung eines Films, wobei das Verfahren die folgenden Schritte umfasst:
(a) Bereitstellen eines wasserlöslichen Filmbildners;
(b) gründliches Einmischen des Filmbildners in eine Fettalkoholkomponente unter Bildung eines Gemisches, das weniger als 5 Gew.-% Wasser hat, wobei das Gemisch die Fettalkoholkomponente in einer Prozentgewichtskonzentration des Gesamtfilms umfasst, die ausreichend ist, um zu bewirken, dass der Film in einem relativ engen Temperaturbereich von 20°C oder weniger von einem Feststoff in eine Flüssigkeit übergeht; und
(c) Extrudieren des Gemisches unter Bildung eines aufgetragenen Films, wobei das Gemisch eine Temperatur von wenigstens 65°C hat, wenn es extrudiert wird.

8. Verfahren gemäß einem der Ansprüche 6 bis 7:
(a) wobei die Fettalkoholkomponente bei einer Temperatur von wenigstens 20°C und weniger als 120°C schmilzt.

9. Verfahren gemäß einem der Ansprüche 6 bis 8:
(a) wobei die Fettalkoholkomponente in einer Prozentgewichtskonzentration des Gesamtfilms vorliegt, die ausreichend ist, um zu bewirken, dass das Gemisch eine Viskosität von 150.000 Centipoise oder weniger hat.

10. Verfahren gemäß einem der Ansprüche 6 bis 9:
(a) wobei die Fettalkoholkomponente einen geradkettigen C₈-C₂₀-Alkohol umfasst.

11. Verfahren gemäß einem der Ansprüche 6 bis 10:
(a) wobei die Fettalkoholkomponente wenigstens einen, ausgewählt aus der Gruppe, bestehend aus Myristylalkohol, Cetylalkohol, Stearylalkohol und Gemischen davon umfasst.

12. Verfahren gemäß einem der Ansprüche 6 bis 11, wobei der Schritt des Einmischens des Filmbildners umfasst:
(a) Einmischen des Filmbildners in flüssigen Stearylalkohol.

13. Verfahren gemäß einem der Ansprüche 6 bis 12:
(a) wobei der wasserlösliche Filmbildner thermoplastisch ist.

14. Verfahren gemäß einem der Ansprüche 6 bis 13, wobei der Schritt des Bereitstellens eines wasserlöslichen Filmbildners umfasst:
(a) Bereitstellen von Hydroxypropylcellulose.

15. Verfahren gemäß einem der Ansprüche 6 bis 14, wobei der Schritt des Extrudierens des Gemisches umfasst:
(a) Extrudieren des Gemisches durch eine Breitschlitzdüse.

16. Verfahren gemäß einem der Ansprüche 6 bis 14, außerdem umfassend die Schritte:
(a) vor einem Extrudieren des Gemisches unter Bildung des aufgetragenen Films, Verfestigen des Gemisches unter Bildung eines festen Materials;
(b) Schmelzen des festen Materials unter Bildung eines geschmolzenen Materials; und
(c) Extrudieren des geschmolzenen Materials.

17. Verfahren gemäß einem der Ansprüche 6 bis 16, außerdem umfassend die Schritte nach Extrudieren des Gemisches:
(a) Wegschneiden eines Teils des aufgetragenen Films an Rändern des aufgetragenen Films;
(b) Schmelzen des weggeschnittenen Teils des aufgetragenen Films unter Bildung eines geschmolzenen Materials; und danach
(c) Extrudieren des geschmolzenen Materials unter Bildung eines zweiten aufgetragenen Films.

18. Verfahren gemäß einem der Ansprüche 6 bis 17, wobei der Schritt des Mischens des Filmbildners mit einer Substanz unter Bildung eines Gemisches umfasst:
(a) Mischen des Filmbildners mit einer Substanz unter Bildung eines Gemisches, das weniger als 2 Gew.-% Wasser hat.

19. Verfahren gemäß einem der Ansprüche 6 bis 18, wobei der Schritt des Mischens des Filmbildners mit einer Substanz unter Bildung eines Gemisches umfasst:
(a) Mischen des Filmbildners mit einer Substanz unter Bildung eines Gemisches, das kein zugesetztes Wasser hat.

20. Film gemäß Anspruch 1, wobei die Fettalkoholkomponente wenigstens 10 Gew.-% des Gesamtfilmgewichts ist.

21. Verfahren gemäß einem der Ansprüche 6 bis 7, wobei die Fettalkoholkomponente wenigstens 10 Gew.-% des Gesamtfilmgewichts ist.

## Revendications

1. Film formé par le procédé comprenant les étapes consistant à :
(a) fournir une matière filmogène hydrosoluble ;
(b) mélanger parfaitement la matière filmogène dans un composant d'alcool gras pour former un mélange, le composant d'alcool gras présent à un niveau de pourcentage en poids du film total suffisant pour faire en sorte que le film se transforme de solide en liquide au sein d'une plage de température relativement étroite de 20°C ou moins ; et
(c) extruder le mélange pour former un film enduit, dans lequel le mélange est à une température d'au moins 65 °C lorsqu'il est extrudé ;
(d) de sorte que, lorsque le film enduit refroidit jusqu'à 30 °C ou moins, le film extrudé ne soit pas poisseux.

2. Film selon la revendication 1, dans lequel :
(a) la matière filmogène comprend de l'alcool stéarique liquide.

3. Film selon la revendication 1, dans lequel :
(a) la matière filmogène hydrosoluble est thermoplastique.

4. Film selon la revendication 1, dans lequel :
(a) la matière filmogène hydrosoluble est de l'hydroxypropylcellulose.

5. Film selon une quelconque des revendications 1 à 4, dans lequel :
(a) le mélange est extrudé à travers une filière à fente.

6. Procédé de fabrication d'un film, le procédé comprenant les étapes consistant à :
(a) fournir une matière filmogène hydrosoluble ;
(b) mélanger parfaitement la matière filmogène dans un composant d'alcool gras pour former un mélange, le composant d'alcool gras présent à un niveau de pourcentage en poids du film total suffisant pour faire en sorte que le film se transforme de solide en liquide au sein d'une plage de température relativement étroite de 20°C ou moins ; et
(c) extruder le mélange pour former un film enduit, dans lequel le mélange est à une température d'au moins 65°C lorsqu'il est extrudé.

7. Procédé de fabrication d'un film, le procédé comprenant les étapes consistant à :
(a) fournir une matière filmogène hydrosoluble ;
(b) mélanger parfaitement la matière filmogène avec un alcool gras pour former un mélange possédant moins de 5 % en poids d'eau, dans lequel le mélange comprend de l'alcool gras à un niveau de pourcentage en poids du film total suffisant pour faire en sorte que le film se transforme de solide en liquide au sein d'une plage de température relativement étroite de 20°C ou moins ; et
(c) extruder le mélange pour former un film enduit, dans lequel le mélange est à une température d'au moins 65 °C lorsqu'il est extrudé.

8. Procédé selon une quelconque des revendications 6 à 7 :
(a) dans lequel le composant d'alcool gras fond à une température d'au moins 20°C et moins de 120 °C.

9. Procédé selon une quelconque des revendications 6 à 8 :
(a) dans lequel le composant d'alcool gras est compris à un niveau de pourcentage en poids du film total suffisant pour faire en sorte que le mélange ait une viscosité de 150000 centipoises ou moins.

10. Procédé selon une quelconque des revendications 6 à 9 :
(a) dans lequel le composant d'alcool gras comprend un alcool C8-C20 à chaîne droite.

11. Procédé selon une quelconque des revendications 6 à 10 :
(a) dans lequel le composant d'alcool gras comprend au moins un élément sélectionné parmi le groupe constitué d'alcool myristique, alcool cétylique, alcool stéarique et des mélanges de ceux-ci.

12. Procédé selon une quelconque des revendications 6 à 11, dans lequel l'étape consistant à mélanger la matière filmogène comprend l'étape consistant à :
(a) mélanger la matière filmogène dans de l'alcool stéarique liquide.

13. Procédé selon une quelconque des revendications 6 à 12, dans lequel :
(a) la matière filmogène hydrosoluble est thermoplastique.

14. Procédé selon une quelconque des revendications 6 à 13, dans lequel l'étape consistant à fournir une matière filmogène hydrosoluble comprend l'étape consistant à :
(a) fournir de l'hydroxypropylcellulose.

15. Procédé selon une quelconque des revendications 6 à 14, dans lequel l'étape consistant à extruder le mélange comprend l'étape consistant à :
(a) extruder le mélange à travers une filière à fente.

16. Procédé selon une quelconque des revendications 6 à 14, comprenant en outre les étapes consistant à :
(a) avant d'extruder le mélange pour former le film enduit, solidifier le mélange pour former une matière solide ;
(b) faire fondre la matière solide pour former une matière fondue ; et
(c) extruder la matière fondue.

17. Procédé selon une quelconque des revendications 6 à 16, comprenant en outre les étapes consistant à, après avoir extrudé le mélange :
(a) ébavurer une partie du film enduit aux bords du film enduit ;
(b) faire fondre la partie ébavurée du film enduit pour former une matière fondue ; et puis
(c) extruder la matière fondue pour former un second film enduit.

18. Procédé selon une quelconque des revendications 6 à 17, où l'étape consistant à mélanger la matière filmogène avec une substance pour former un mélange comprend l'étape consistant à :
(a) mélanger la matière filmogène avec une substance pour former un mélange possédant moins de 2 % en poids d'eau.

19. Procédé selon une quelconque des revendications 6 à 18, où l'étape consistant à mélanger la matière filmogène avec une substance pour former un mélange comprend l'étape consistant à :
(a) mélanger la matière filmogène avec une substance pour former un mélange ne comprenant pas d'eau ajoutée.

20. Film selon la revendication 1, dans lequel le composant d'alcool gras est au moins 10 % en poids par poids de film total.

21. Procédé selon une quelconque des revendications 6 à 7, dans lequel le composant d'alcool gras est au moins 10 % en poids par poids de film total.
